# EUROPEAN PATENT SPECIFICATION

(11) **EP 2 046 817 B1**
(45) Date of publication and mention of the grant of the patent: **16.12.2009**
(21) Application number: 07799327.7
(22) Date of filing: 05.07.2007
(51) Int. Cl.: C07K 14/00, C08G 69/10

(54) **IMPROVED PROCESS FOR THE PREPARATION OF COPOLYMER-1**
VERBESSERTES VERFAHREN ZUR HERSTELLUNG VON COPOLYMER-1
PROCÉDÉ AMÉLIORÉ DE PRÉPARATION DE COPOLYMÈRE-1

(30) Priority: 05.07.2006 US 806585 P
(43) Date of publication of application: 15.04.2009
(60) Divisional application: 09174515.8
(73) Proprietor: Momenta Pharmaceuticals, Inc., Cambridge, MA 02142 (US)
(72) Inventor: IYER, Mani, S., North Chemsford, MA 01863 (US); BAUER, Corinne, Sudbury, MA 01176 (US); OLIVER-SHAFFER, Pat, Acton, MA 01720 (US)
(74) Representative: Peterreins, Frank
(86) International application number: PCT/US2007/072865
(87) International publication number: WO 2008/006026

(56) References cited:
- WO-A-95/31990
- WO-A-2004/043995
- WO-A-2006/050122
- D TEITELBAUM ET AL.: "Suppression of experimental allergic encephalomyelitis by a synthetic polypeptide" EUROPEAN JOURNAL OF IMMUNOLOGY, vol. 1, 1971, pages 242-248, XP002926270 DE WEINHEIM

## Description

### TECHNICAL FIELD

The presently disclosed subject matter generally relates to processes for polymerizing amino acids. More particularly, the presently disclosed subject matter relates to processes for preparing copolymer-1.

### BACKGROUND

Copolymer-1 is a complex mixture of polypeptides prepared from the polymerization of the amino acids glutamic acid, lysine, alanine and tyrosine. Copolymer-1 also is known as glatiramer acetate and has the following structural formula:

(Glu, Ala, Lys, Tyr)ₓ XCH₃COOH

(C₅H₉NO₄•C₃H₇NO₂•C₆H₁₄N₂O₂•C₉H₁₁NO₃)ₓ•XC₂H₄O₂

Glatiramer acetate (GA) is the active ingredient of COPAXONE® (Teva Pharmaceutical Industries Ltd., Israel), which comprises the acetate salts of synthetic polypeptides containing four naturally occurring amino acids: L-glutamic acid, L-alanine, L-tyrosine, and L-lysine, with a reported average molar fraction of 0.141, 0.427, 0.095, and 0.338, respectively.
Glatiramer synthesis is i.a. described in WO-A-2006050/22 (Novartis) or in Eur. J. Immunol. 1, 1971, page 242-248.

Glatiramer acetate is used in the treatment of the relapsing-remitting form of multiple sclerosis (RRMS).

### BRIEF SUMMARY

The presently disclosed subject matter provides methods for preparing copolymer-1. In one embodiment, a mixture ofN-carboxyanhydrides (NCAs) comprising alanine, protected glutamic acid, protected lysine, and tyrosine, wherein the protected glutamic acid includes a benzyl or a methoxy protecting group and the protected lysine includes a cyclic imide protecting group, are contacted with a polymerization initiator to initiate polymerization of the mixture of N-carboxyanhydrides to form a protected copolymer-1. The protected copolymer-1 can then be treated with one or more deprotecting reagents and/or depolymerized to obtain a copolymer-1. In one embodiment, the method includes deprotecting the protected glutamic acid and protected lysine to produce copolymer-1.

In some embodiments, the method further comprises measuring the molecular weight of the copolymer-1 as the polymerization of the mixture of N-carboxyanhydrides is proceeding. Measuring the molecular weight as the polymerization is proceeding can permit the polymerization reaction or, in some embodiments, the depolymerization reaction to be terminated when the copolymer-1 has obtained a desired molecular weight. In one embodiment, the molecular weight can be determined by one or more of an in-line method, an on-line method, an off-line method, and combinations thereof. In one embodiment, the molecular weight can be determined by using an infrared (IR) probe to measure the amount of a carbamate moiety (as determined by the intensity of one or more infrared absorption bands characteristic of the carbamate moiety) in the reaction mixture as the polymerization reaction is proceeding. The measured amount of the carbamate moiety can then be correlated to a model that relates molecular weight as a function of the amount of the carbamate moiety present in the reaction mixture. In another embodiment, an amount of an amide carbonyl moiety can be measured during the depolymerization reaction. The measured amount of the amide carbonyl moiety can be correlated to a model that relates molecular weight as a function of the amount of the amide carbonyl moiety present in the reaction mixture.

The presently disclosed methods overcome many of the problems that can be associated with prior art methods for preparing a polypeptide, such as copolymer-1. More particularly, the presently disclosed methods can be more efficient and can permit the synthesis of copolymer-1 having a desired molecular weight.

Certain aspects of the presently disclosed subject matter having been stated hereinabove, which are addressed in whole or in part by the presently disclosed subject matter, other aspects will become evident as the description proceeds when taken in connection with the accompanying Examples and Figures as best described herein below.

### BRIEF DESCRIPTION OF THE FIGURES

Having thus described the presently disclosed subject matter in general terms, reference will now be made to the accompanying figures, which are not necessarily drawn to scale, and wherein:
Figure I is a representative, non-limiting reaction scheme depicting a presently disclosed method for preparing copolymer-1, wherein glutamic acid is protected with a methoxy protecting group and lysine is protected with a Boc protecting group; and

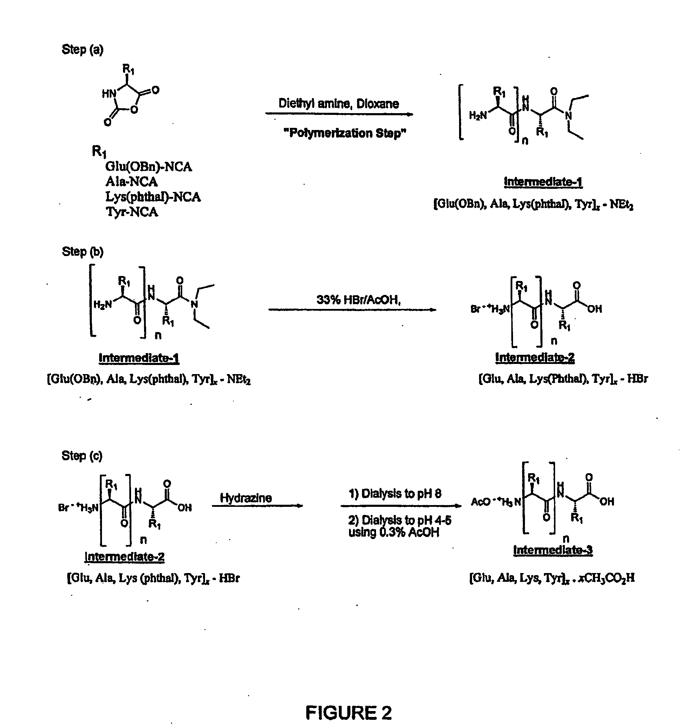

Figure 2 is a representative, non-limiting reaction scheme depicting a presently disclosed method for preparing copolymer-1, wherein glutamic acid is protected with a benzyl protecting group and lysine is protected with a phthaloyl protecting group.

### DETAILED DESCRIPTION

The presently disclosed subject matter now will be described more fully hereinafter with reference to the accompanying figures, in which some, but not all embodiments of the presently disclosed subject matter are shown. Many modifications and other embodiments of the presently disclosed subject matter set forth herein will come to mind to one skilled in the art to which the presently disclosed subject matter pertains having the benefit of the teachings presented in the foregoing descriptions and the associated figures. Therefore, it is to be understood that the presently disclosed subject matter is not to be limited to the specific embodiments disclosed and that modifications and other embodiments are intended to be included within the scope of the appended claims. Although specific terms are employed herein, they are used in a generic and descriptive sense only and not for purposes of limitation.

The terms "a," "an," and "the" refer to "one or more" when used in this application, including the claims. Thus, for example, reference to "a sample" includes a plurality of samples, unless the context clearly is to the contrary (e.g., a plurality of samples), and so forth.
All publications, patent applications, patents, and other references are herein incorporated by reference to the same extent as if each individual publication, patent application, patent, and other reference was specifically and individually indicated to be incorporated by reference. It will be understood that, although a number of patent applications, patents, and other references are referred to herein, such reference does not constitute an admission that any of these documents forms part of the common general knowledge in the art.

Throughout the description, where compositions are described as having, including, or comprising specific components, or where processes or methods are described as having, including, or comprising specific steps, it is contemplated that compositions of the presently disclosed subject matter also can consist essentially of, or consist of the recited components, and that the processes or methods of the presently disclosed subject matter also consist essentially of or consist of the recited steps. Further, it should be understood that the order of steps or order for performing certain actions are immaterial so long as the presently disclosed subject matter remains operable. Moreover, two or more steps or actions can be conducted simultaneously with respect to the presently disclosed subject matter disclosed herein.

In some embodiments, the presently disclosed subject matter provides a method for preparing a synthetic polypeptide, such as copolymer-1, in which a mixture ofN-carboxyanhydrides (NCAs) having protected and non-protected amino acids are polymerized to form a polypeptide. As used herein, a "polypeptide" refers to a polymer comprising amino acid residues that are bonded together with amide linkages, which are commonly referred to as peptide bonds. The peptide bond is formed from a bond between a carbonyl group on the C-terminus end of an amino acid and the nitrogen group on the N-terminus end of another amino acid. When many amino acids are linked using these peptide linkages they form polypeptides.

A polypeptide can include a polymer made from the same amino acids, different amino acids, or combinations thereof. Polypeptides can have a random ordering to the amino acids or ordering to the amino acids. In some embodiments, the polypeptide can be a polymer comprised only of L amino acids or D amino acids, or some combination thereof in any proportion. More particularly, the phrase "a polypeptide mixture," refers to, in some embodiments, a mixture of copolymers of the amino acids comprising L-glutamic acid, L-alanine, L-tyrosine, and L-lysine, or analogs or derivatives thereof.

As used herein, the terms "copolymer", "amino acid copolymer" or "amino acid copolymer preparation" refer to a heterogeneous mixture of polypeptides consisting of a defined plurality of different amino acids (typically between 2-10, e.g., 2, 3, 4, 5, 6, 7, 8, 9, or 10 different amino acids, and, in some embodiments, between 3-6, e.g., 3, 4, 5, or 6, different amino acids). A copolymer, as described herein, can be prepared from the polymerization of individual amino acids. The term "amino acid" is not limited to naturally occurring amino acids, but can include amino acid derivatives and/or amino acid analogs. For example, in an amino acid copolymer comprising tyrosine amino acids, one or more of the amino acids can be a homotyrosine. Further, an amino acid copolymer having one or more non-peptide or peptidomimetic bonds between two adjacent residues is included within this definition. A copolymer can be non-uniform with respect to the molecular weight of each species of polypeptide within the mixture. A specific copolymer according to the presently disclosed subject matter comprises a mixture of polypeptides comprising alanine (A), glutamic acid (E), lysine (K), and tyrosine (Y). Accordingly, in one embodiment, the copolymer comprises a mixture of polypeptides consisting of the amino acids Y, E, A, and K and also is referred to as Copolymer 1 (Cop 1) or glatiramer acetate (GA).

More particularly, in some embodiments, the presently disclosed subject matter provides a method for preparing copolymer-1, the method comprising contacting a mixture of N-carboxyanhydrides (NCAs) having the following formula: 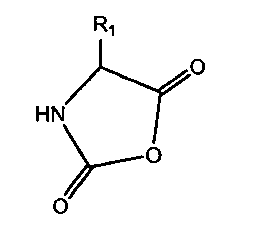 wherein R₁ comprises one or more amino acids selected from the group consisting of alanine, protected glutamic acid, protected lysine, and tyrosine, with a polymerization initiator to initiate polymerization of the mixture ofNCAs comprising one or more amino acids to produce a protected copolymer-1 having the following structure: 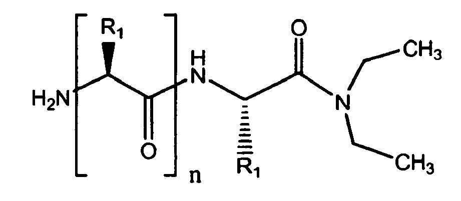 wherein R₁ is the same as defined above and n is an integer depicting a polymeric chain comprising amide linkages.

In subsequent steps, the protecting groups can be removed from the protected glutamic acid and protected lysine to produce copolymer-1. In one embodiment, the presently disclosed methods can be used to prepare copolymer-1 having a molecular weight between about 2 kDa to about 40 kDa. In other embodiments, the presently disclosed methods can be used to prepare copolymer-1 having a molecular weight between about 2 kDa and about 25 kDa; in some embodiments, between about 4 kDa to about 16 kDa; in some embodiments, between about 4 kDa to about 9 kDa; in some embodiments, between about 4 kDa to about 8 kDa; in some embodiments, between about 9 kDa to about 12 kDa; in some embodiments, between about 10 kDa and about 11 kDa; and, in some embodiments, between about 10 kDa to about 12 kDa. As used herein, the phrase "molecular weight" means peak average molecular weight (Mp). The molecular weight of copolymer 1 can be measured, for example, by gel permeation chromatography (GPC) using, for example, a SUPEROSE 12™ or SUPERDEX™ column calibrated with protein standards, polymethylmethacrylate (PMMA) standards, or other appropriate standards known and available in the art. A heterogeneous mixture of polypeptides such as copolymer-1 may also be described by other metrics known in the art, including but not limited to the weight average molecular weight (Mw), the number average molecular weight (Mn), the z-average molecular weight (Mz), and the polydispersity of the polypeptide mixture.

Suitable polymerization initiators can include, for example, bases, nucleophiles, and combinations thereof. In one embodiment, the polymerization initiator can include one or more amines, alcohols, water, and combinations thereof. In one embodiment, the polymerization initiator comprises one or more secondary amines. Suitable secondary amines include, but are not limited to, dimethylamine, diethylamine, di-n-propylamine, di-isopropylamine, N-ethylmethylamine, di-n-butylamine, di-iso-butylamine, di-sec-butylamine, di-tert-buylamine, diamylamine, di-n-octylamine, di-(2-ethylhexyl)-amine, diisononylamine, diallylamine, N-methylaniline, diphenylamine, aziridine, pyrrole, pyrrolidine, imidazole, indole, piperidine, purine, and combinations thereof. Other polymerization initiators can include K-tOBu, NaH, KH, triethylamine (TEA), tetramethyl piperdine, dicyclohexylamine, dicyclohexylundecane (DCU), lithiumdiisopropyl amine, t-BuLi, and combinations thereof.

Contacting the polymerization initiator with the mixture of N-carboxyanhydrides begins the propagation of the amino acids and the synthesis of copolymer-1. In one embodiment, the molecular weight of the copolymer-1 can be controlled by terminating the polymerization reaction when the molecular weight of the copolymer-1 is within a desired range, e.g., from about 10 kDa to about 12 kDa.

In one embodiment, the method for preparing copolymer-1 includes contacting a mixture ofNCAs with a polymerization initiator, wherein the mixture ofNCAs includes glutamic acid having a benzyl or methoxy (OMe) protecting group. In yet another embodiment, the mixture of NCAs includes a lysine having a cyclic imide derivative or a t-butoxycarbonyl (Boc) protecting group. Cyclic imide derivatives suitable for use with the presently disclosed methods include, but are not limited to, phthalimide, *N-*tetrachlorophthalimide, 4-Nitro-*N*- phthalimide, *N*-dithiasuccinimide, *N*-2,3-diphenylmaleimide, *N*-2,5-dimethylpyrrole, N-2,5-bis(triisopropylsiloxy)pyrrole, *N*-1,1,4,4-tetramethyldisilylazacyclopentane adduct, and the like. In some embodiments, the lysine protecting group is phthalimide.

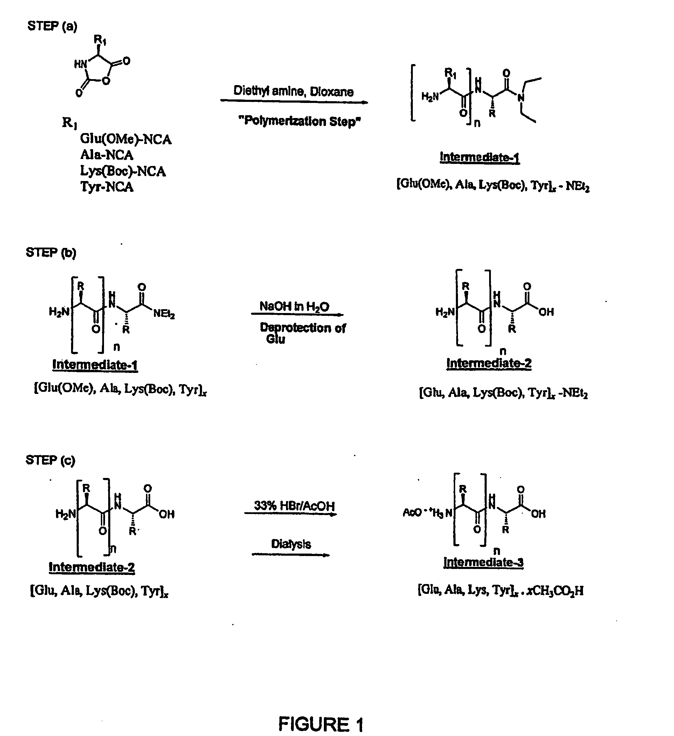

More particularly, in one embodiment, copolymer-1 can be prepared by contacting a mixture of *N*-carboxyanhydrides (NCAs) having the following structure: 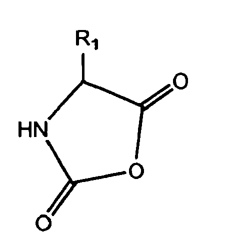 wherein R₁ comprises one or more amino acids selected from the group consisting of alanine, methoxy-protected glutamic acid, Boc-protected lysine, and tyrosine, with a polymerization initiator to form a protected copolymer-1. Referring now to Figure 1, an exemplary multi-step reaction scheme for preparing copolymer-1 is provided. In step (a), a mixture ofNCAs is contacted with a polymerization initiator, such as diethylamine, to form protected copolymer-1 intermediate **1.** In this example, glutamic acid is protected with a methoxy group and lysine is protected with a Boc group. Step (a) can be carried out in variety of solvents, including, but not limited to, tetrahydrofuran (THF), dichloromethane (DCM), dioxane, N-methylpyrrolidone (NMP), dimethylformamide (DMF), and acetonitrile (ACN), for example.

In step (b), intermediate **1** is treated with a deprotecting reagent, such as NaOH, that deprotects the methoxy-protected glutamic acid. The reaction can then be neutralized with an acid, such as HBr, to produce intermediate **2.** In step (c), intermediate **2** is treated with an HBr/acetate mixture to remove the Boc-lysine protecting group, thereby producing copolymer-1, labeled as intermediate **3** in Figure 1.

The resulting copolymer-1 can then be purified and characterized using known methods, for example, lyophilization and/or dialysis to produce a salt of copolymer-1. It should be recognized that in some embodiments the order of step (b) and step (c) can be reversed. For example, in one embodiment, intermediate 1 can be treated with HBr in glacial acetic acid, to remove the Boc-lysine protecting group, followed by treating the resulting copolymer-1 intermediate with a base, such as NaOH, to remove the glutamic acid methoxy protecting group.

Referring now to Figure 2, an alternate reaction scheme for preparing copolymer-1 is illustrated in a step-wise manner. In this embodiment, glutamic acid is protected with a benzyl group and lysine is protected with a phthalimide protecting group. In step (a), a mixture of NCAs is contacted with a polymerization initiator, such as diethylamine, to form protected copolymer-1 intermediate 1. The polymerization step, i.e., step (a) of Figure 2, can be carried out in variety of solvents, including, but not limited to, THF, DCM, dioxane, NMP, DMF, and ACN, for example.

Referring once again to Figure 2, in step (b), intermediate 1 is treated with a deprotecting reagent, such as HBr in glacial acetic acid, to remove the benzyl protecting group from the protected glutamic acid to form intermediate **2.** In step (c), intermediate **2** is treated with a deprotecting reagent that removes the phthalimide protection group from lysine to produce an unprotected copolymer-1. Suitable deprotecting reagents that can be used to remove the phthalimide protecting group include, but are not limited to, hydrazine, phenyl hydrazine, methyl amine, butyl amine, sodium hydroxide, hydroxyl amine-sodium methoxide, and hydrazine acetate. The resulting unprotected copolymer-1 can then be purified and characterized using known methods, for example, lyophilization and/or dialysis to produce a salt of copolymer-1.

In one embodiment, a high purity acid is used in the process of preparing copolymer-1. In one embodiment, a high purity acid comprises less than 0.5% of free halogen and less than 1000 ppm of metal ion impurities.

After removing any protecting groups that can be present, the copolymer-1 can be further purified. Suitable methods of purifying the copolymer-1 can include, but are not limited to, dialysis, vacuum desiccation, lyophilization, recrystallization, refluxing, various forms of chromatography, precipitation methods, sublimation methods, filtration, adductive, extractive and melt crystallization, evaporation, solid/liquid separations, centrifugation, column separation processes, distillation, azeotropic, extractive and steam distillation, ion exchange methods, membrane separation techniques, adsorption separation processes, simulated moving bed chromatography, solid/liquid extraction and leaching, liquid/liquid extraction, and supercritical fluid extraction, and combinations thereof.

In one embodiment, once the polymerization reaction is quenched, the product of the reaction can be purified using chromatography methods such as GPC. The GPC column can be packed with, for example, SUPERDEX™ ((available from GE Healthcare BioSciences Corp., Piscataway, New Jersey) or its equivalent, and/or SUPEROSE™ (GE Healthcare) or its equivalent.

Any mobile phase commonly used in the art can be employed in the GPC separation method including aqueous and organic solvents and any combination thereof. In one non-limiting embodiment, the copolymer-1 can be dissolved in the mobile phase and then eluted through a calibrated, packed GPC column. Protein standards, PMMA standards, or other appropriate standards known and available in the art may be used to calibrate the GPC column. A detector, e.g., an ultraviolet (UV) detector, can be used to determine when the copolymer-1 elutes through the column. Fractions of the eluted copolymer-1 are collected and the molecular weight can be determined from the elution time.

Other purification methods that can be used to purify copolymer-1 according to the presently disclosed methods include, but are not limited to, vacuum desiccation; lyophilization; recrystallization; extractions, refluxing; precipitation methods; sublimation methods; filtration; adductive, extractive and melt crystallization; evaporation; solid/liquid separations; centrifugation; column separation processes; sedimentation; distillation; azeotropic, extractive and steam distillation; ion exchange methods; membrane separation techniques; adsorption separation processes; simulated moving bed chromatography; solid/liquid extraction and leaching; liquid/liquid extraction; supercritical fluid extraction; capillary electrophoresis (CE), including Capillary Zone Electrophoresis (CZE), Capillary Gel Electrophoresis (CGE), Capillary Isoelectric Focusing (CIEF), Isotachophoresis (ITP), Electrokinetic Chromatography (EKC), Micellar Electrokinetic Capillary Chromatography (MECC OR MEKC), Micro Emulsion Electrokinetic Chromatography (MEEKC), NonAqueous Capillary Electrophoresis (NACE), and Capillary Electrochromatography (CEC); Gel electrophoresis; dialysis; HPLC; RP-HPLC; affinity chromatography; gas chromatography, including gas-liquid chromatography, gas-solid chromatography, partition chromatography, adsorption chromatography, thin-layer chromatography, and supercritical fluid chromatography. In general, any of the techniques disclosed immediately hereinabove can be used alone or in combination and in any order to purify copolymer-1.

Contacting the polymerization initiator with the mixture of N-carboxyanhydrides initiates the propagation of the amino acids and thereby begins the synthesis of copolymer-1. In one embodiment, the molecular weight of the copolymer-1 can be controlled by terminating the polymerization reaction when the molecular weight of the copolymer-1 is within a desired range, e.g., from about 10 kDa to about 12 kDa.

Accordingly, in one embodiment, the presently disclosed method for preparing copolymer-1 can include measuring the molecular weight of the copolymer product as the polymerization reaction is proceeding. In such embodiments, measuring the molecular weight as the polymerization reaction is proceeding can permit the reaction to be stopped when the copolymer-1 has reached a desired molecular weight. In this way, a copolymer-1 having a desired molecular weight range can be prepared. As a result, the need for additional steps such as depolymerization to obtain a desired molecular weight can be reduced or, in some cases, eliminated.

In one embodiment, the molecular weight can be determined by removing sample aliquots of the copolymer as the polymerization reaction is proceeding. The molecular weight of the copolymer can then be determined using known methods, including, but not limited to, GPC and other methods of chromatography using proteins, PMMA, or other appropriate standards known and available in the art to calibrate the column, end group analysis, vapor phase methods, elevation of boiling points method, ebulliometry, osmotic pressure, use of a prinner-stabin osometer, diffusion and gradient methods, light scattering method, solution viscometry methods, IR methods, and X-ray methods.

As discussed further herein below, in some embodiments, the molecular weight can be determined by introducing an in-line instrument, such as an infrared probe, into the reaction mixture. The in-line instrument can be used to determine the molecular weight of the copolymer-1 *in situ* as the polymerization reaction is proceeding. Measuring the molecular weight of the copolymer-1 as the reaction is proceeding can permit termination of the reaction once the copolymer-1 has obtained a desired molecular weight. As a result, a copolymer-1 having a desired molecular weight can be obtained in the absence of using a reagent, such as HBr, that cleaves the polypeptide intermediate into smaller molecular weight segments.

The polymerization reaction can be terminated with any of a number of methods that are known in the art. For example, in one embodiment, the reaction can be terminated by adding a sufficient amount of water to quench the polymerization reaction. In some embodiments, the polymerization reaction can be terminated when the copolymer-1 has a molecular weight between about 2 kDa and about 40 kDa. In another embodiment, the polymerization reaction can be terminated when the copolymer-1 has a molecular weight exceeding about 3 kDa; in some embodiments, about 4 kDa; in some embodiments, about 5 kDa; in some embodiments, about 6 kDa; in some embodiments, about 7 kDa; in some embodiments, about 8 kDa; in some embodiments, about 9 kDa; and, in some embodiments, about 10 kDa. In yet another embodiment, the polymerization reaction can be terminated when the copolymer-1 has a molecular weight less than about 25 kDa; in some embodiments, less than about 20 kDa; in some embodiments, less than about 15 kDa; in some embodiments, less than about 14 kDa; in some embodiments, less than about 13 kDa; in some embodiments, less than about 12 kDa; and, in some embodiments, less than about 11 kDa. In one embodiment, the molecular weight of the copolymer-1 is between about 10 kDa to about 12 kDa.

In some embodiments, the molecular weight of copolymer-1 can be determined as the polymerization reaction is proceeding by using an in-line method, an on-line method, an off-line method, and combinations thereof. As used herein, the term "in-line" refers to a method for measuring the molecular weight of the copolymer-1 as the polymerization reaction is proceeding by introducing a measuring device or probe directly into the reaction vessel. For example, in one embodiment, the molecular weight of the copolymer-1 can be obtained by introducing an IR probe into the reaction vessel during the polymerization reaction. In one embodiment, the IR probe can measure an amount of carbamate present during the polymerization reaction, for example, as determined by the intensity of characteristic carbamate IR absorption bands. Generally, the amount of carbamate, and the intensity of its IR absorption bands, should decrease as the polymerization reaction is proceeding. The decrease in the amount of carbamate in the reaction mixture should be proportional to an increase in the molecular weight of copolymer-1. The change in IR intensity of the characteristic carbamate absorption bands can be correlated to a model that depicts molecular weight as a function of the amount of carbamate present in the mixture. From this model, the molecular weight of the copolymer-1 can be determined at any point during the polymerization reaction.

In other embodiments, the IR probe can be used to measure the intensity of infrared absorption bands characteristic of an amide carbonyl moiety present during depolymerization, the intensity of which increases as the molecular weight decreases. The amide carbonyl IR intensity also can be correlated to a model that depicts molecular weight as a function of the amount of the amide carbonyl moiety present in the mixture. The measured intensity can then be used to stop either the polymerization or depolymerization reaction when the copolymer-1 is within a desired molecular weight range. Other methods of measuring the amount, e.g., the concentration of, carbamate and/or amide carbonyl moieties in the mixture can include Raman, ultra-violet, other vibrational techniques, and similar spectral analysis techniques.

In addition to measuring the amount of carbamate and/or amide carbonyl moieties present in the mixture as a method for determining the molecular weight in the reaction mixture, other methods can be used to correlate reaction progress to the molecular weight of the copolymer. Such other methods include, but are not limited to, viscosity measurements, turbidity measurements, CO₂ measurements, density and dilatormetry measurements, ultrasonic measurements, fluorescence spectroscopy, calorimetry measurements, and the like. For example, in one embodiment, the amount of CO₂ generated during the polymerization reaction can be correlated to the molecular weight of the copolymer-1. CO₂ is generated during the polymerization reaction as the NCAs are consumed. In other embodiments, the measured viscosity and/or turbidity of the copolymer-1 can be correlated to a model that depicts molecular weight as a function of polymer viscosity and/or turbidity.

In other embodiments, the molecular weight of the copolymer-1 can be determined by in an "off-line" method. In an off-line method, aliquots of the polymerization mixture are removed from the reaction vessel as the reaction is proceeding. The reaction is quenched in the aliquot and the molecular weight can be determined using various methods, such as using a GPC column calibrated with proteins, PMMA, or other appropriate standards known and available in the art and the like. In one embodiment, this off-line method also can include slowing down the polymerization reaction in the reactor vessel during the period of time in which the molecular weight is being determined off-line. For example, the reaction can be slowed by cooling the reaction vessel to a temperature between 0 °C and 10 °C.

In another embodiment, the molecular weight can be determined "on-line" during the course of the polymerization reaction. In this embodiment, the reaction vessel can include a channel through which an aliquot of the reaction mixture can be temporarily cycled out of the reaction vessel for molecular weight determination and then reintroduced to the reaction vessel. For example, in one embodiment, the reactor can include a channel that has two or more openings that are capable of being in fluid communication with the interior of the reaction vessel. The openings can include a gate or similar device, such as a valve, that can prevent the ingress or egress of the reaction mixture into the channel. At a desired time, one of the gates or valves can be opened to permit an aliquot of the reaction mixture to flow into the channel. The second gate or valve at an opposite end of the channel can remain in a closed position. The sample can then be analyzed to determine the molecular weight of the reaction mixture. The aliquot can then be returned to the reaction mixture by opening the second gate or valve. In this embodiment, techniques such as those discussed hereinabove in relation to determining molecular weight using in-line techniques, including, but not limited to, IR, Raman, ultraviolet spectra analysis, can be used. In some embodiments, the sample aliquot also can be permanently removed from the reaction vessel.

In one alternative embodiment, a NCA or mixture ofNCAs can be prepared by reacting one or more amino acids having the following structure: 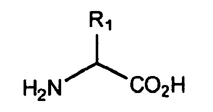 wherein R₁ is the same as described above, with phosgene or a phosgene surrogate. Suitable phosgene surrogates include, but are not limited to, diphosgene, triphosgene, carbonyl diimidazole, disuccinimidyl carbonate, and combinations thereof.

In some embodiments, the ratio of D, L stereoisomers present in the polypeptide can be selectively controlled. The stereochemistry of the resulting peptide linkage can be controlled by the stereoisometry of the amino acids that are used to synthesize the polypeptide. In contrast, activated amino acids having a ring structure can result in a mix of dextrorotatory (D) and levorotatory (L) stereoisomers. In one embodiment, the polypeptide can comprise from about 80 percent to about 100 percent L enantiomers and from about 0 percent to about 20 percent D enantiomers. In another embodiment, the polypeptide can comprise greater than about 75, 80, 85, 90, 95, 96, 97, 98, or 99 percent L enantiomers. In yet another embodiment, the polypeptide can have less than about 100, 99, 98, 97, 96, 95, 90, 85, or 80 percent L enantiomers.

In another non-limiting embodiment, the copolymer-1 can comprise the acetate salts of synthetic polypeptides containing four naturally occurring amino acids: L-glutamic acid, L-alanine, L-tyrosine, and L-lysine with an average molar fraction of about 0.141, 0.427, 0.095, and 0.338, respectively. The molecular weight of the copolymer-1 can be between about 4,700 to about 11,000 Daltons. In one embodiment, the chemical formula of the copolymer-1 is L-glutamic acid polymer with L-alanine, L-lysine and L-tyrosine, acetate (salt). Its structural formula can be represented as: (Glu, Ala, Lys, Tyr)ₓ XCH₃COOH or [(C₅H₉NO₄ C₃H₇NO₂ C₆H₁₄NO₂ C₉H₁₁NO₃)ₓXC₂H₄O₂].

In yet another non-limiting embodiment, the copolymer-1 can be added to a pharmaceutically acceptable excipient and is formed as a white to off-white, sterile, lyophilized powder containing between about 50 mg to about 10 mg of glatiramer acetate and between about 100 mg to about 10 mg of mannitol. In another embodiment, the copolymer-1 can be added to a pharmaceutically acceptable excipient and can be formed as a white to off-white, sterile, lyophilized powder containing about 20 mg of glatiramer acetate and about 40 mg of mannitol. In one embodiment, the copolymer-1 with the pharmaceutically acceptable excipient can be supplied in single-use or multiple-use vials and can be administered using subcutaneous administration after reconstitution with any diluent supplied, e.g., sterile water for injection.

### EXAMPLES

The following Examples have been included to provide guidance to one of ordinary skill in the art for practicing representative embodiments of the presently disclosed subject matter. In light of the present disclosure and the general level of skill in the art, those of skill can appreciate that the following Examples are intended to be exemplary only and that numerous changes, modifications, and alterations can be employed without departing from the scope of the presently disclosed subject matter. Thus, the following Examples are offered by way of illustration and not by way of limitation.

### EXAMPLE I

### Preparation of copolymer-1 Using Methoxy-Protected Glutamic Acid and Boc-Protected Lysine

**Step (a):** Referring now to Scheme 1, in a first step, a mixture of N-carboxyanhydrides of tyrosine (3 g, 14.5 mmol, 1.0 eq), alanine (8.3 g, 72.1 mmol, 5.0 eq), γ-methoxyglutamate (5.8 g, 22.2 mmol, 1.5 eq), ε-*N*-Boc-lysine (13.8 g, 51.5 mmol, 3.6 eq) is charged to a 1.0-L jacketed flask under a small stream of nitrogen. The flask includes a mechanical stirrer and a temperature probe. 583.3 mL of dioxane is then added to the flask. The reaction mixture is stirred for 30 min, to which 116 µL (1.1 mmol, 0.08 eq) of diethyl amine (polymerization initiator) is added to the flask using a pipette. The reaction mixture turns viscous and very cloudy over the first hour. 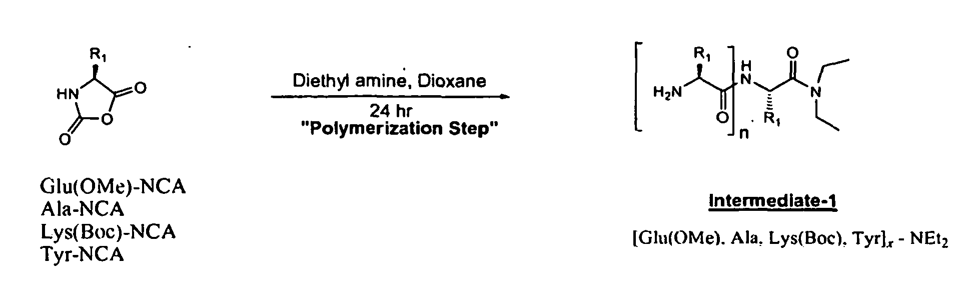 Scheme 1. Polymerization of a mixture of NCAs to form a protected copolymer-1.

After about 24 hours the reaction mixture is quenched by pouring the mixture into a second flask (3.0 L) containing water (1.58 L) with vigorous stirring. A white colored solid is formed. The precipitate is isolated by vacuum filtration and washed with water (6 x 250 mL), then dried overnight to constant weight in a vacuum oven to obtain intermediate **1.**

**Step (b)**: Referring now to Scheme 2, in the following step, the methoxy protecting group is removed from the protected glutamic acid by treating intermediate 1 with NaOH. In this step, 1.8 g of intermediate 1 is charged to a 250-mL flask. Aqueous one molar sodium hydroxide is added to the flask and is stirred for 24 hours at a temperature ranging between 4 °C and 15 °C. After the reaction is complete, the reaction mixture is neutralized to pH = 7 using aqueous HBr. The solids are filtered and dried in a vacuum oven to obtain intermediate **2** ([Glu, Ala, Lys(Boc), Tyr]x).

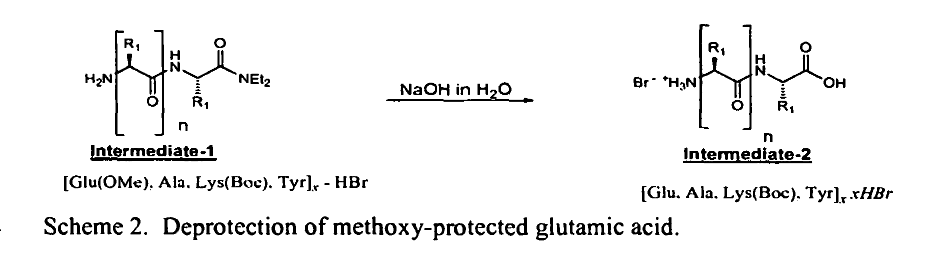

**Step (c)**: Referring now to Scheme 3, in this step, 22 g of intermediate **2** is charged to a 500-mL jacketed flask having a mechanical stirrer. To this, 177.2 mL of 33% HBr in acetic acid is added and stirred at a temperature of about 20 °C for about 20 to 30 hours. The resulting reaction mixture is dialyzed and lyophilized to obtain intermediate **3** as a white to off-white colored solid.

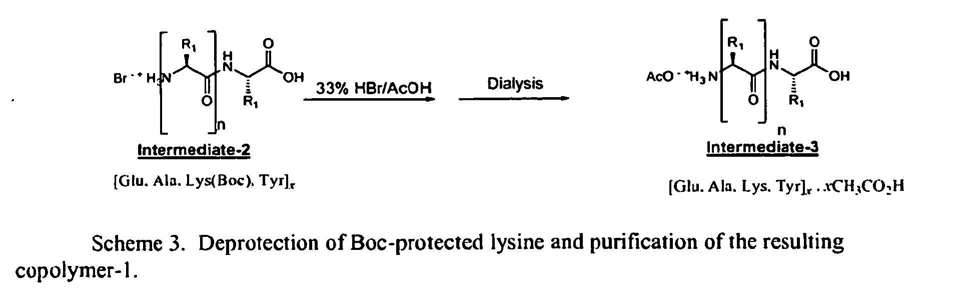

### EXAMPLE 2

### Preparation of Copolymer-1 Using Benzyl-Protected Glutamic Acid and Phthaloyl-Protected Lysine

Referring now to Scheme 3, under a small stream of nitrogen, a mixture of N-carboxyanhydrides of tyrosine (3 g, 14.5 mmol, 1.0 eq), alanine (8.3 g, 72.1 mmol, 5.0 eq), γ-Bn-glutamate (5.8 g, 22.2 mmol, 1.5 eq), ε-N-phthaloyl-lysine (13.8 g, 51.5 mmol, 3.6 eq) is charged to a 1.0-L jacketed flask. The flask includes a mechanical stirrer and a temperature probe. 583.3 mL of dioxane is then added to the flask. The reaction mixture is stirred for 30 min, to which 116 µL (1.1 mmol, 0.08 eq) of diethyl amine (polymerization initiator) is added to the flask using a pipette. The reaction mixture turns viscous and very cloudy over the first hour. After about 24 hours, the reaction mixture is quenched by pouring it into another flask (3.0 L) containing water (1.58 L) and with vigorous stirring. A white colored solid is formed. The precipitate is isolated by vacuum filtration and washed with water (6 x 250 mL). The resulting product is dried overnight to constant weight in a vacuum oven to obtain intermediate **1**.

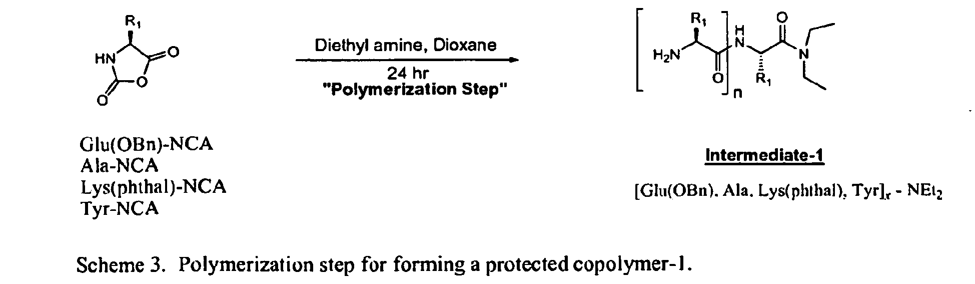

Referring now to Scheme 4, charge 22 g of intermediate 1 to a 500-mL jacketed flask having a mechanical stirrer. To this mixture 177.2 mL of 3% HBr in acetic acid is added and stirred at a temperature of about 20 °C for about 20 to 30 hours. The reaction is quenched by transferring the reaction mixture to a flask (stirred with a mechanical stirrer) containing 660 mL of water. The solid is vacuum filtered and washed with 3 x 40 mL of water and 3 x 40 mL of diisopropyl ether. The solid is dried over night in a vacuum oven (at 37 °C) to obtain intermediate **2** as a white-colored solid. 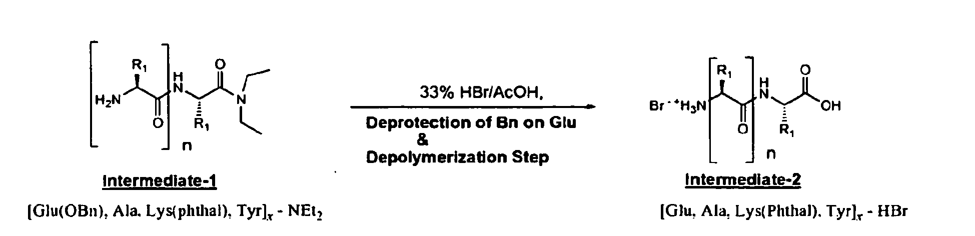 Scheme 4. Deprotection of benzyl-protected glutamic acid.

Referring now to Scheme 5, charge 1.8 g of intermediate **2** to a 250-mL flask. To this add hydrazine solution. The mixture is stirred for a period of time between 15-24 hours. The reaction mixture is filtered to remove any fine insoluble material and the filtrate is passed through an ultra-filtration using a 1 KD membrane first with circulating water until pH 8 is observed in the permeate and then circulating with 0.3% acetic acid in water to pH 5.5-6.0 in the retentate. The solution is then lyophilized to obtain intermediate **3** as a white to off-white solid.

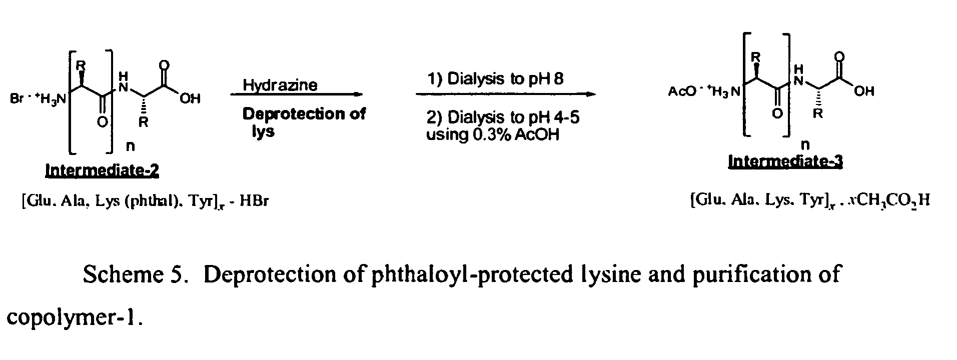

Many modifications and other embodiments of the presently disclosed subject matter set forth herein will come to mind to one skilled in the art to which the presently disclosed subject matter pertains having the benefit of the teachings presented in the foregoing descriptions and the associated figures. Therefore, it is to be understood that the presently disclosed subject matter is not to be limited to the specific embodiments disclosed and that modifications and other embodiments are intended to be included within the scope of the appended claims.

## Claims

1. A method for preparing copolymer-1, the method comprising:
(a) polymerizing a mixture of the N-carboxyanhydride of alanine, the N-carboxyanhydride of tyrosine, the N-carboxyanhydride of benzyl or methoxy protected glutamic acid, and the N-carboxyanhydride of a cyclic imide protected lysine by contacting the mixture with a polymerization initiator to produce protected copolymer-1;
(b) deprotecting the protected glutamic acid and the protected lysine to produce copolymer-1.

2. The method of claim 1 further comprising measuring the molecular weight of the copolymer-1.

3. The method of claim 1 or claim 2 wherein the cyclic imide protected lysine is selected from: phthalimide protected lysine, N-tertrachlorophthalimide protected lysine, 4-Nitro-N-phthalimide protected lysine, N-dithaisuccinimide protected lysine, N-2,3-diphenylmaleimide protected lysine, N-2.5-dimethylpyrrole protected lysine, N-2,5-bis(triisonpropylsiloxy)pyrrole protected lysine, and tetrametlyldisilylazecyclopentane protected lysine.

4. The method of claim 3 wherein the cyclic imide protected lysine is phthalimide protected lysine.

5. The method of any of claims 1 - 4 wherein the mixture comprises the N-carboxyanhydride of benzyl protected glutamic acid or wherein the mixture comprises the N-carboxyanhydride of methoxy protected glutamic acid.

6. The method of any of claim 1 - 3 wherein the polymerization initiator comprises one or more secondary amines selected from the group consisting of dimethylamine, diethylamine, di-n-propylamine, di-isopropylamine, N-ethylmethylamine, di-n-butylamine, di-iso-butylamine, di-sec-butylamine, di-tert-buylamine, diamylamine, di-n-octylamine, di-(2-ethylhexyl)-amine, diisononylamine, diallylamine, N-methylaniline, diphenylamine, aziridine, pyrrole, pyrrolidine, imidazole, indole, piperidine, purine, and combinations thereof.

7. The method of claim 4 wherein the phthalimide protected lysine is deprotected by reaction with an agent selected from the group consisting of: hydrazine, phenyl hydrazine, methyl amine, butyl amine, sodium hydroxide, hydroxyl amine-sodium methoxide and hydrazine acetate.

8. The method of claim 1 wherein the protected glutamic acid is deprotected before the protected lysine is deprotected or wherein the protected lysine is deprotected before the protected glutamic is deprotected.

9. The method of claim 1 wherein molar ratio of alanine:glutamic acid:lysine:tyrosine in the copolymer-1 is about 5-7:1-3:4-6:2-2.2, optionally wherein molar ratio of alanine:glutamic acid:lysine:tyrosine in the copolymer-1 is about 6:2:5:1.

10. The method of claim 2 wherein the average molecular weight of the copolymer-1 is between about 2 kDa and about 40 kDa.

11. The method of claim 1 further comprising purifying the copolymer-1.

12. The method of claim 2 wherein the step of measuring the molecular weight of the protected copolymer-1 occurs in situ.

13. The method of claim 2 wherein the step of measuring the molecular weight of the protected copolymer-1 comprises chromatography.

14. The method of claim 2 wherein the step of measuring the molecular weight of the protected copolymer-1 comprises measuring the amount of carbamate or an amide carbonyl moiety in the polymerization mixture.

15. The method of claim 1 further comprising purifying the copolymer-1 and combining the purified copolymer-1 with a pharmaceutically acceptable excipient.

## Patentansprüche

1. Verfahren zur Hestellung von Copolymer-1, wobei das Verfahren umfasst:
(a) Polymerisieren einer Mischung des N-Carboxyanhydrid von Alanin, des N-Carboxyanhydrid von Tyrosin, des N-Carboxyanhydrid von durch Benzyl oder eine Methoxygruppe geschützter Glutaminsäure, und des N-Carboxyanhydrid von durch zyklisches Imid geschütztem Lysin, durch Kontaktieren der Mischung mit einem Polymerisations-Initiator, um das geschützte Copolymer-1 zu erzeugen;
(b) Abspalten der Schutzgruppe der geschützten Glutaminsäure und des geschützten Lysins, um das Copolymer-1 zu erzeugen.

2. Verfahren gemäß Anspruch 1, des Weiteren umfassend Messen des Molekulargewichts des Copolymer-1.

3. Verfahren gemäß Anspruch 1 oder Anspruch 2, wobei das durch zyklisches Imid geschützte Lysin ausgewählt ist aus: durch Phthalimid geschütztes Lysin, durch N-Tertrachlorophthalimid geschütztes Lysin, durch 4-Nitro-N-phthalimid geschütztes Lysin, durch N-dithaisuccinimid geschütztes Lysin, durch N-2,3-diphenylmaleimid geschütztes Lysin, durch N-2,5-dimethylpyrrol geschütztes Lysin, durch N-2,5-bis(triisonpropylsiloxy)pyrrol geschütztes Lysin, und durch Tetramethyldisilylazacyclopentan geschütztes Lysin.

4. Verfahren gemäß Anspruch 3, wobei das durch zyklisches Imid geschützte Lysin durch Phthalimid geschütztes Lysin ist.

5. Verfahren gemäß einem der Ansprüche 1 bis 4, wobei die Mischung das N-Carboxyanhydrid von durch Benzyl geschützter Glutaminsäure umfasst oder wobei die Mischung das N-Carboxyanhydrid von durch eine Methoxygruppe geschützter Glutaminsäure umfasst.

6. Verfahren gemäß einem der Ansprüche 1 bis 3, wobei der Polymerisations-Initiator umfasst eines oder mehrerer sekundäre Amine ausgewählt aus der Gruppe bestehend aus: Dimethylamin, Diethylamin, Di-n-propylamin, Diisopropylamin, N-Ethylmethylamin, Di-n-butylamin, Di-iso-butylamin, Di-sec-butylamin, Di-tert-buylamin, Diamylamin, Di-n-octylamin, Di-(2-ethylhexyl)-amin, Di-isononylamin, Diallylamin, N-Methylanilin, Diphenylamin, Aziridin, Pyrrol, Pyrrolidin, Imidazol, Indol, Piperidin, Purin, und Kombinationen aus diesen.

7. Verfahren gemäß Anspruch 4, wobei die Schutzgruppe des durch Phthalimid geschützten Lysin abgespalten wird durch eine Reaktion mit einem Stoff ausgewählt aus der Gruppe bestehend aus: Hydrazin, Phenylhydrazin, Methylamin, Butylamin, Natriumhydroxid, Hydroxylamin-Natriummethoxid und Hydrazinazetat.

8. Verfahren gemäß Anspruch 1, wobei die Schutzgruppe der geschützten Glutaminsäure abgespalten wird bevor die Schutzgruppe des geschützten Lysins abgespalten wird oder wobei die Schutzgruppe des geschützten Lysins abgespalten wird bevor die Schutzgruppe der geschützten Glutaminsäure abgespalten wird.

9. Verfahren gemäß Anspruch 1, wobei das molare Verhältnis von Alanin:Glutaminsäure:Lysin:Tyrosin in dem Copolymer-1 ungefähr 5-7:1-3:4-6:2-2,2 ist, optional wobei das molare Verhältnis von Alanin:Glutaminsäure:Lysin:Tyrosin in dem Copolymer-1 ungefähr 6:2:5:1 ist.

10. Verfahren gemäß Anspruch 2, wobei das durchschnittliche Molekulargewicht des Copolymer-1 zwischen ungefähr 2 kDa und ungefähr 40 kDa liegt.

11. Verfahren gemäß Anspruch 1, des Weiteren Reinigen des Copolymer-1 umfassend.

12. Verfahren gemäß Anspruch 2, wobei der Schritt des Messens des Molekulargewichts des geschützten Copolymer-1 in situ geschieht.

13. Verfahren gemäß Anspruch 2, wobei der Schritt des Messens des Molekulargewichts des geschützten Copolymer-1 Chromatographie umfasst.

14. Verfahren gemäß Anspruch 2, wobei der Schritt des Messens des Molekulargewichts des geschützten Copolymer-1 das Messen der Menge von Carbamat oder eines Amidcarbonylrests in der Polymerisations-Mischung umfasst.

15. Verfahren gemäß Anspruch 1, des Weiteren umfassend Reinigen des Copolymer-1 und Kombinieren des Copolymer-1 mit einem pharmazeutisch zulässigen Hilfsstoff.

## Revendications

1. Procédé de préparation d'un copolymère-1, le procédé comprenant :
(a) la polymérisation d'un mélange du N-carboxyanhydride d'alanine, du N-carboxyanhydride de tyrosine, du N-carboxyanhydride de l'acide glutamique protégé par un benzyle ou un méthoxy, et du N-carboxyanhydride d'une lysine protégée par un imide cyclique en mettant en contact le mélange avec un initiateur de polymérisation pour produire un copolymère-1 protégé ;
(b) la déprotection de l'acide glutamique protégé et de la lysine protégée pour produire le copolymère-1.

2. Procédé selon la revendication 1, comprenant en outre la mesure du poids moléculaire du copolymère-1.

3. Procédé selon la revendication 1 ou la revendication 2, dans lequel la lysine protégée par un imide cyclique est choisie parmi : la lysine protégée par un phtalimide, la lysine protégée par un N-tétrachlorophtalimide, la lysine protégée par un 4-nitro-N-phtalimide, la lysine protégée par un N-dithiasuccinimide, la lysine protégée par un N-2,3-di-phénylmaléimide, la lysine protégée par un N-2,5-di-méthylpyrrole, la lysine protégée par un N-2,5-bis(triisopropylsiloxy)pyrrole, et la lysine protégée par un tétraméthyldisilylazacyclopentane.

4. Procédé selon la revendication 3, dans lequel la lysine protégée par un imide cyclique est la lysine protégée par un phtalimide.

5. Procédé selon l'une quelconque des revendications 1 à 4, dans lequel le mélange comprend le N-carboxyanhydride de l'acide glutamique protégé par un benzyle ou dans lequel le mélange comprend le N-carboxyanhydride de l'acide glutamique protégé par un méthoxy.

6. Procédé selon l'une quelconque des revendications 1 à 3, dans lequel l'initiateur de polymérisation comprend une ou plusieurs amines secondaires choisies dans le groupe constitué par la diméthylamine, la diéthylamine, la di-n-propylamine, la di-isopropylamine, la N-éthylméthylamine, la di-n-butylamine, la di-iso-butylamine, la di-sec-butylamine, la di-tert-butylamine, la diamylamine, la di-n-octylamine, la di-(2-éthylhexyl)amine, la diisononylamine, la diallylamine, la N-méthylaniline, la diphénylamine, l'aziridine, le pyrrole, la pyrrolidine, l'imidazole, l'indole, la pipéridine, la purine et leurs combinaisons.

7. Procédé selon la revendication 4, dans lequel la lysine protégée par un phtalimide est déprotégée par réaction avec un agent choisi dans le groupe constitué par : l'hydrazine, la phénylhydrazine, la méthylamine, la butylamine, l'hydroxyde de sodium, l'hydroxylamine-méthoxyde de sodium et l'acétate d'hydrazine.

8. Procédé selon la revendication 1, dans lequel l'acide glutamique protégé est déprotégé avant la déprotection de la lysine protégée ou dans lequel la lysine protégée est déprotégée avant la déprotection de l'acide glutamique protégé.

9. Procédé selon la revendication 1, dans lequel le rapport molaire alanine : acide glutamique : lysine : tyrosine dans le copolymère-1 est d'environ 5 à 7 : 1 à 3 : 4 à 6:2 à 2,2, facultativement dans lequel le rapport molaire alanine : acide glutamique : lysine : tyrosine dans le copolymére-1 est d'environ 6 : 2 : 5 : 1.

10. Procédé selon la revendication 2, dans lequel le poids moléculaire moyen du copolymère-1 se situe entre environ 2 kDa et environ 40 kDa.

11. Procédé selon la revendication 1, comprenant en outre la purification du copolymère-1.

12. Procédé selon la revendication 2, dans lequel l'étape de mesure du poids moléculaire du copolymère-1 protégé est réalisée *in situ*.

13. Procédé selon la revendication 2, dans lequel l'étape de mesure du poids moléculaire du copolymère-1 protégé comprend la chromatographie.

14. Procédé selon la revendication 2, dans lequel l'étape de mesure du poids moléculaire du copolymère-1 protégé comprend la mesure de la quantité de carbamate ou d'un fragment carbonyle d'amide dans le mélange de polymérisation.

15. Procédé selon la revendication 1, comprenant en outre la purification du copolymère-1 et la combinaison du copolymère-1 purifié avec un excipient pharmaceutiquement acceptable.
